# EUROPEAN PATENT SPECIFICATION

(11) **EP 1 858 445 B1**
(45) Date of publication and mention of the grant of the patent: **14.09.2016**
(21) Application number: 06738302.6
(22) Date of filing: 14.03.2006
(51) Int. Cl.: A61F 2/915

(54) **CRACK/FATIGUE RESISTANT ENDOPROSTHESIS**
BRUCH-/ERMÜDUNGSRESISTENTE ENDOPROTHESE
ENDOPROTHESE RESISTANT AUX CRAQUELURES ET/OU A LA FATIGUE

(30) Priority: 14.03.2005 US 661542 P; 13.03.2006 US 374923; 13.03.2006 US 375380; 13.03.2006 US 375381
(43) Date of publication of application: 28.11.2007
(73) Proprietor: ABBOTT LABORATORIES, Abbott Park, IL 60064-3500 (US)
(72) Inventor: VON OEPEN, Randolf, Los Altos Hills, California 94024 (US); YRIBARREN, Travis, R., San Mateo, California 94401 (US); CLIFFORD, Anton,G., Mountain View, California 94040 (US)
(74) Representative: Boult Wade Tennant
(86) International application number: PCT/US2006/009227
(87) International publication number: WO 2006/099450

(56) References cited:
- EP-A- 0 875 218
- WO-A-03/037221
- US-A1- 2004 102 837
- US-A1- 2004 186 554
- US-B1- 6 402 777

## Description

### 1. The Field of the Invention

The present invention relates to an endoprosthesis for delivery and deployment within a body vessel of a human or animal. More particularly, the invention relates to an endoprosthesis with improved crack and/or fatigue resistance.

### 2. The Relevant Technology

Stents, grafts, and a variety of other endoprostheses are well known and used in interventional procedures, such as for treating aneurysms, for lining or repairing vessel walls, for filtering or controlling fluid flow, and for expanding or scaffolding occluded or collapsed vessels. Such endoprostheses can be delivered and used in virtually any accessible body lumen of a human or animal, and can be deployed by any of a variety of recognized means. One recognized indication of endoprostheses, such as stents, is for the treatment of atherosclerotic stenosis in blood vessels. For example, after a patient undergoes a percutaneous transluminal coronary angioplasty or similar interventional procedure a stent is often deployed at the treatment site to improve the results of the medical procedure and to reduce the likelihood of restenosis. The stent is configured to scaffold or support the treated blood vessel. If desired, a stent can also be loaded with a beneficial agent so as to act as a delivery platform to reduce restenosis or for other beneficial purposes.

An endoprosthesis is typically delivered by a catheter delivery system to a desired location or deployment site inside a body lumen of a vessel or other tubular organ. To facilitate such delivery, the endoprosthesis can be capable of having a particularly small cross-sectional profile to access deployment sites within small diameter vessels. Additionally, the intended deployment site may be difficult to access by a physician and can involve traversing the delivery system through a tortuous luminal pathway. Thus, it can be desirable to provide the endoprosthesis with a sufficient degree of flexibility during delivery to allow advancement through the anatomy to the deployed site. Moreover, it can be desirable for the endoprosthesis to have sufficient crack and/or fatigue resistance so as to retain structural integrity during and/or after being deployed and set.

Generally, an endoprosthesis can-be constructed of multiple annular members or rings which are interconnected either through a connection section or a connection element. Accordingly, flexibility of the endoprosthesis can be controlled by the number and/or width of the rings, the characteristics of connection sections or elements, and/or the thickness of material that forms the rings. Although it is not specifically known how much vessel restenosis can be attributed to stent rigidity, it is know that a reasonably stiff stent may injure the vessel during motion (e.g., vessel contraction and/or expansion during pulsatile blood flow). As such, it can be desirable for an endoprosthesis to have sufficient flexibility/stiffness properties to enable deployment through a tortuous luminal pathway. Also, it can be desirable to change the stiffness properties of the endoprosthesis after deployment within a vessel. However, it can also be important for the endoprosthesis to retain its structural integrity after deployment by being configured to inhibit the formation and/or propagation of cracks as well as resist structural fatigue.

Once deployed, the endoprosthesis can be capable of satisfying a variety of performance characteristics. The endoprosthesis can be sufficiently rigid or provide an outwardly-oriented bias when deployed to perform its intended function, such as opening a lumen or supporting a vessel wall. Similarly, the endoprosthesis can have suitable flexibility along its length and/or width to inhibit any kinking or straightening that may occur during deployment or setting within the tortuous luminal pathway.

In WO 03/037221 there is described a stent with a plurality of relief cuts formed In the interconnected struts. The relief cuts are formed to extend through the struts and be filled with plugs of therapeutic material, where one or both exterior surfaces of the stent are covered with a medicine or other type of coating. Multiple coatings may also be applied to the stent. In particular coatings may be applied to some regions of the stent and not be present on other regions. The relief cuts are placed at flexion points, such that the struts have increased widths and reduced thicknesses while lattice support relief cuts between flexion points Increase adhesion of the therapeutic coating on the stent.

A significant failure mode in endoprostheses can be as a result of crack formation and/or propagation through the body of an endoprosthesis. For example, failure can result from a stent element, such as a strut or elbow, beginning to crack during deployment, and propagation of the crack during setting and use. Such cracks can also form and/or propagate through the material of the endoprosthesis as a result of the cyclic loading that the stent undergoes during the pulsatile movement of blood and associated vessel expansion and contraction. For example, endoprosthetic fatigue-induced failures can be encountered when nitinol stents are used in the superficial femoral artery ("SFA").

An additional problem with existing endoprosthesis designs can be related to the difficulty in properly placing the endoprosthesis within a vessel prior to deployment. Current endoprosthesis designs have thinner struts that utilize less radiopaque material, and therefore may not be as visible under fluoroscopy. An attempt to address the reduced radiopacity can include the use of at least one radiopaque marker band disposed on the delivery device and or endoprosthesis, wherein the marker band may be utilized to indicate an end of the endoprosthesis device, a length, a width, or the like. Other methods of increasing the radiopacity of an endoprosthesis can include the addition of radiopaque markers either disposed upon a surface of the endoprosthesis or within a retaining member associated therewith. One shortcoming of some present designs relates to the difficulty in manufacturing, and therefore increased costs. Also, due to size limitations of the radiopaque material used, the markers may not provide sufficient visibility for precise placement.

Although various endoprostheses have been developed to address one or more of the aforementioned performance shortcomings, there remains a need for a more versatile design that improves one or more performance characteristics without sacrificing the remaining characteristics. Therefore, it would be advantageous to have an endoprosthesis configured to have increased resistance to cracking or fatiguing during deployment, setting, and/or use. Additionally, it would be beneficial for the endoprosthesis to have sufficient strength and flexibility to enable deployment through tortuous luminal pathways while retaining the ability to perform its intended function.

### SUMMARY OF THE INVENTION

The present invention relates to an endoprosthesis for delivery into a lumen of a body. The endoprosthesis is configured to have improved crack and/or fatigue resistance so as to retain substantial structural integrity during and/or after deployment. The improvement in structural integrity inhibits crack formation and/or propagation and/or fatigue-induced catastrophic failure, thereby improving the performance and reliability of the endoprostheses. The inhibition of catastrophic failure prevents portions of the endoprostheses from puncturing the lumen or damaging the tissue of the lumen. Thus, inhibiting crack formation and/or propagation and/or fatigue-induced catastrophic failure improves the safety and longevity of endoprostheses.

According to the present invention there is provided an endoprosthesis for delivery in a body lumen, the endoprosthesis comprising: at least one strut element having a strut width and strut thickness, the strut thickness being defined by a first side opposite a second side, the strut element having a plurality of holes extending from the first side to the second side in a random arrangement that extends from a first end to a second end of the strut element to inhibit crack propagation.

The at least one strut element may be defined by an element width, a thickness, and a length, wherein the length can have a substantially straight, arcuate, rounded, curved or other similar-shape. The strut element thickness can be defined by a first side that is opposite a second side, wherein both may be orthogonal with the width and/or length. As such, the cross-sectional profile of the strut element can range from planar, rounded, circular, square, rectangular, triangular, octagonal, polygonal, or other possible shapes. The strut element has a plurality of holes that extend from the first side to the second side of the strut thickness; however, the holes can extend through any dimension of the strut element that is not substantially congruent with the length. The plurality of holes are arranged so as to inhibit crack propagation across the strut element length, width, and/or thickness. The plurality of holes inhibits a strut element from incurring catastrophic failure that breaks the element into more than one piece.

The endoprosthesis can include a set of interconnected strut elements that define an annular element. Substantially each strut element can be defined by a body having various width, thickness, and length characteristics. That is, the body can have different configurations dependent on the shape of the cross-sectional profile and shape of the length. Any of the bodies can have at least a first portion connected to a second portion; however, any number of portions can be interconnected as needed. Generally, two adjacent portions are connected through a plurality of connecting members so that failure of a single connecting member does not compromise the overall integrity of the strut member or endoprosthesis. For example, the adjacent portions can be connected by a first outer connecting member, middle connecting member, and second outer connecting member, wherein the first outer connecting member and the middle connecting member define a first hole and the second outer connecting member and the middle connecting member define a second hole. Also, additional connecting members can be used to connect adjacent portions of a strut body. The connecting members can each have a cross-sectional area and/or shape that cooperate with the material thereof to provide sufficient strength so that at least two of the connecting members can provide structural integrity to the strut member. That is, only two connecting members are needed for the endoprosthesis to retain sufficient functionality. The first hole, second hole, and any other additional hole formed by the interconnected connecting members can have a wall having a shape that inhibits crack formation and/or propagation, wherein rounded, circular, arcuate, straight, bent, shapes substantially devoid of sharp angles, and the like are representative of shapes that can inhibit crack formation and/or propagation. Hole walls that are susceptible to crack formation and/or propagation can be characterized by having at least one angle or sharp angle that can stress the connecting members.

In one embodiment, the present invention can additionally include a fiber or particle reinforced endoprosthesis. Such an endoprosthesis includes at least one strut element comprised of a matrix including a primary material and a plurality of fibers or particles. Usually, the primary material is a shape memory material or other material to form a self-expanding or balloon-expanding endprosthesis. The fibers or particles are dispersed within the primary material in an amount and distribution that inhibits crack propagation.

In one embodiment, the present invention includes an endoprosthesis that is structurally resistant to cracking by including holes and either fiber, or particles. Accordingly, the endoprosthesis includes at least one strut element having a strut width and a strut thickness. The strut thickness is defined by a first side opposite a second side, where the strut element has a plurality of holes that extend from the first side to the second side along the strut width. The strut element is comprised of a matrix including a primary material and a plurality of fibers or particles. The fibers or particles are dispersed within the primary material in an amount and distribution to inhibit crack propagation.

There is also described a method of manufacturing an endoprosthesis for delivery in a body lumen that has improved resistance to crack propagation and/or fatigue-induced failure. The method includes forming a tubular structure comprised of a first material. The tubular structure has a cylindrical shape with an internal lumen, wherein the tubular structure has a sufficient length and wall thickness for processing. The tubular structure is processed so as to form a plurality of holes in the tubular structure. Also, the tubular structure is shaped into a shape of an endoprosthesis. The hole-forming and shaping can be performed in any order or simultaneously. The shaping can be performed by cutting a first portion of the first material from the tubular structure so as to form a first side of a plurality of interconnected strut elements, and subsequently cutting a second portion of the first material so as to form a second side of the plurality of interconnected strut elements. The shaping and/or hole formation can define size and shape of the interconnected strut elements that form an annular element. The shaped and/or hole-forming can be by any process that can penetrate through the thickness of the material of the tubular structure before or after the interconnected strut elements are formed.

There is also described a method of making an endoprosthesis without the endoprosthesis needing to be further processed or shaped. However, further processing and shaping can always be performed. Such an improved method of making an endoprosthesis can optionally include sintering. Accordingly, the method can include the following: providing a mold having a shape of an endoprosthesis; injecting sinterable particles into a mold; molding the sinterable particles into a green body; and sintering the green body to obtain the endoprosthesis.

A similar method of making an endoprosthesis can also include sintering. Such a method can include the following: providing a mold having a shape of an endoprosthesis, wherein the mold contains a plurality of sinterable particles; and sintering the sinterable particles to obtain an endoprosthesis with at least one strut element.

These and other features of the present invention will become more fully apparent from the following description, drawings, and/or appended claims, or may be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only some embodiments of the invention and are therefore not to be considered limiting of its scope. The invention will be described and explained with additional specificity and detail through the use of the accompanying drawings, in which:
Figure 1 is a planar side view of an exemplary endoprosthesis in accordance with the invention;
Figures 2A-2B are planar side views of compacted endoprostheses in accordance with the present invention;
Figure 3 is a perspective view of a deployed tubular endoprosthesis in accordance with the present invention;
Figures 4A-4D show are schematic diagrams of strut elements having holes;
Figures 5A-5E are cross-sectional profiles of strut elements having holes;
Figure 6 depicts cross-sectional profiles of strut elements;
Figures 7A-7E are cross-sectional profiles of holes within an endoprosthetic element;
Figures 8A-8B are volumetric profiles illustrating cubic strut elements having holes;
Figures 9A-9B are volumetric profiles illustrating planar strut elements having holes; and
Figures 10A-10D are volumetric profiles illustrating embodiments of planar strut elements having fiber-reinforced matrices.

### DETAILED DESCRIPTION OF THE PREFERRED EMBODIMENTS

The present invention includes various embodiments of endoprostheses for delivery into a lumen of a body of a human or other animal. The endoprostheses are configured to have improved crack and/or fatigue resistance by including crack propagation-inhibiting holes that extend through the endoprosthetic body. Additionally, the holes can increase the ability of the endoprostheses to sustain cyclic loading without suffering fatigue related failures. Thus, the endoprostheses having the holes can retain substantial structural integrity during and/or after deployment.

The improvement in endoprosthetic structural integrity from strut elements having holes can inhibit crack formation and/or propagation and/or fatigue-induced catastrophic failure, thereby improving the performance and reliability of the endoprostheses. The inhibition of catastrophic failure can prevent portions of the endoprostheses from puncturing the lumen or damaging the tissue of the lumen. Thus, inhibiting crack formation and/or propagation and/or fatigue-induced catastrophic failure can improve the safety and longevity of endoprostheses.

As used herein, the term "catastrophic failure" is meant to refer to a section of an endoprosthesis breaking into multiple portions. For example, catastrophic failure of an elbow can result in a high area snapping into two pieces. Also, it can result in portions of the endoprosthesis coupled by the elbow to become uncoupled, where the ends of the break can adversely interact or puncture adjacent luminal tissue.

Generally, improvements in structural integrity can be accomplished by an endoprosthetic element having a matrix that includes crack-inhibiting features. Such crack-inhibiting features can serve to terminate crack propagation through the endoprosthetic element. Moreover, such crack-inhibiting features can reduce crack formation and/or crack propagation and/or fatigue-induced catastrophic failure. Some examples of crack-inhibiting features can include holes, apertures, recesses, fibers, particles, voids, pockets, cavities, and the like.

The holes in the strut element can serve multiple purposes, including prevention of crack propagation. For example, if a crack begins at the edge of a strut, as it propagates, it will encounter and break into a hole. The hole can then prevent further propagation. In addition, the holes may also create a stronger structure since the material can behave similar to a plurality of interconnected flexible columns.

### I. Endoprosthesis

An endoprosthesis can have various configurations. Examples of some endoprostheses can include stents, filters, grafts, valves, occlusive devices, trocars, aneurysm treatment devices, or the like. Additionally, an endoprosthesis can be configured for a variety of intralumenal applications, including vascular, coronary, biliary, esophageal, urological, gastrointestinal, nasal or the like. While various embodiments of endoprostheses and endoprosthetic elements are described in more detail below, it should be recognized that the embodiments are not limiting and the principles of the present invention can extend to other embodiments of endoprostheses.

In accordance with the present invention, an endoprosthesis having a specific pattern will be described. It shall be understood that the following description of the endoprosthesis should not be considered limiting in any manner and that the present invention is independent of the endoprosthesis pattern. For example, it is contemplated that the present invention may be practiced in accordance with endoprosthesis patterns having: connection sections, connectors; open cell patterns, close cell patterns and the like.

Generally, an endoprosthesis of the present invention includes at least a first set of interconnected strut elements that cooperatively define an annular element. Usually, each strut element can be defined by a cross-sectional profile having a width and a thickness, and include a first end and a second end bounding a length. The length can be characterized as being substantially linear, arced, rounded, squared, other configurations and/or combinations thereof. The strut element can have improved structural integrity by including a plurality of holes extending through the strut element in an amount, distribution, pattern, shape, and/or configuration that can inhibit crack formation and/or propagation and/or fatigue-induced catastrophic failure. The strut element can include a crossbar, connector, elbow, foot, ankle, toe, heel, medial segment, lateral segment, combinations thereof, or the like, as described in more detail below.

Usually, the annular elements can include a plurality of circumferentially-adjacent crossbars that are interconnected end-to-end by an elbow connection or a foot extension. As such, at least one annular element can include an elbow or a foot extension ("foot") extending between at least one pair of circumferentially-adjacent crossbars. The elbow or foot can thus define an apex between the pair of circumferentially-adjacent crossbars of the annular element. Any of the crossbars, elbows, and/or foot elements can include holes that inhibit crack formation and/or propagation and/or fatigue-induced catastrophic failure.

An elbow can be configured in any shape that connects adjacent ends of circumferentially-adjacent crossbars, and can be described as having a U-shape, V-shape, L-shape or the like. The foot can have a foot shape having a first foot portion extending circumferentially from an end of one of the adjacent strut members and at least a second foot portion extending circumferentially from a corresponding end of the other of the circumferentially-adjacent strut members. In combination, the first and second foot portions can generally define an ankle portion connected to a toe portion through a medial segment and the toe portion connected to a heel portion through a lateral segment. As stated above, the present invention will be described in accordance with a specific endoprosthesis design; this should not be considered limiting in any manner. The concepts described herein in accordance with the present invention may be applied to other endoprosthesis designs wherein, in accordance with those designs, the placement of the certain features may differ from that shown or described herein.

In one embodiment, an endoprosthesis can include two or more interconnected annular elements. As such, the endoprosthesis can include at least a second set of interconnected strut elements defining at least a second annular element. Also, the endoprosthesis can include additional annular elements defined by interconnected strut elements as described herein or well known in the art. Each annular element can generally define a ring-like structure extending circumferentially about a longitudinal or central axis. The cross-sectional profile of each annular element can be arcuate, circular, helical, spiral, or the like, although alternative cross-sectional profiles, such as oval, oblong, rectilinear or the like, can be used.

In one embodiment, a first annular element can be aligned longitudinally adjacent to a second annular element along the longitudinal axis, and connected to each other through at least one connection element extending therebetween. The connector element can be considered to be a strut element for the purposes of the invention, and can have a plurality of holes therein. As such, the connector element can be a strut element that interconnects adjacent annular elements, and has improved structural integrity by having holes in an amount, distribution, pattern, shape, and/or configuration to inhibit crack formation and/or crack propagation and/or fatigue-induce catastrophic failure.

Preferably, the first and second annular elements generally define a tubular structure. For example, each annular element can define a continuous closed ring such that the longitudinally-aligned annular elements form a closed tubular structure having a central longitudinal axis. Alternatively, each annular element can define an open ring shape such that the longitudinally-aligned elements form a rolled sheet, open tubular, or "C-shape" type structure. That is, the annular element is not required to be closed. Furthermore, each annular element can define substantially a 360 degree turn of a helical pattern or spiral, such that the end of one annular element can be joined with the corresponding end of a longitudinally-adjacent annular element to define a continuous helical pattern along the length of the endoprosthesis. Moreover, various other annular and endoprosthetic shapes and configurations can be employed within the scope of the present invention by one of ordinary skill in the art.

Each strut crossbar of the annular elements can include a first end and a second end. The crossbar of each annular element can be disposed circumferentially adjacent to each other, and interconnected through elbow elements, foot elements, and/or the like so as to define an expandable structure. For example, and with reference to the closed tubular structure described above, circumferentially-adjacent crossbars of each annular element can be interconnected, either directly or indirectly, in an end-to-end format by an elbow, foot, and/or the like-to define a continuous ring having a generally circular cross-sectional profile. By altering the angle or distance defined between circumferentially-adjacent crossbars, the tubular structure can be radially expanded between a delivery configuration and a deployed configuration. As discussed in detail below, the expandable structure can be expanded by the application of an external force, such as by a balloon, or by a change in delivery conditions, such as an increase in temperature or the removal of a restraint, so as to allow the structure to self expand.

One or more of the strut elements of an annular element include a plurality of holes that prevent crack formation and/or crack propagation and/or fatigue-induced catastrophic failure. Preferably, most or all of the strut elements can include such holes. A section of any particular strut element can have at least one hole, but preferably two or more holes along any particular width that extends through the thickness of the strut body. This can be exemplified by a cross-sectional profile of the strut element being partitioned into two or more discrete sections that are separated by a transverse gap, wherein the transverse gap is a hole that extends through the element. While the present invention is described and illustrated with the width of a strut element having holes that extend through the thickness, it should be recognized that the holes can extend between any points around the perimeter of a cross-sectional slice of the strut element. Also, the holes can have any orientation or angle with respect to the length and/or cross-section of the strut element. Further, some holes or recesses can extend at least partially through the strut element. In still other configurations, the holes can follow a curvilinear path or some other orientated path.

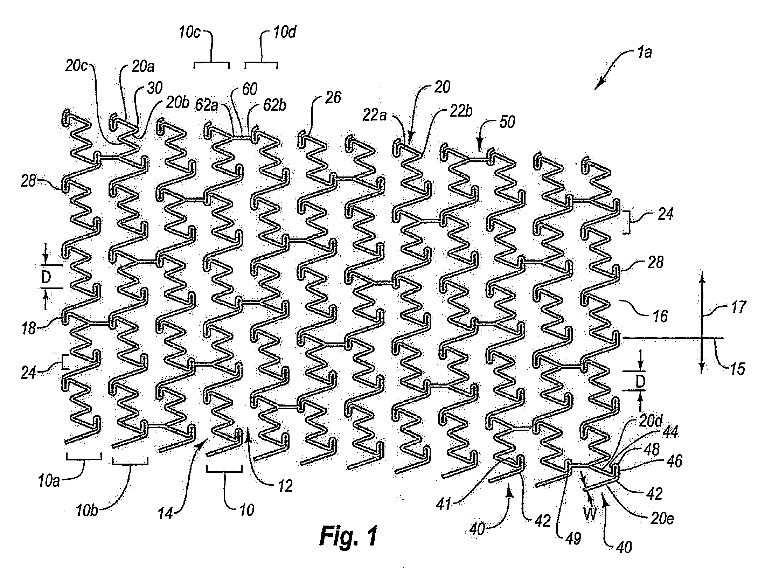

Figure 1 is a side view of a flattened portion of an embodiment of an endoprosthesis 1 a that includes a plurality of holes 26 therein. For purpose of illustration and not limitation, the representative endoprosthesis 1 a is depicted in a deployed configuration and in a planar format for the sake of clarity. As shown, the endoprosthesis 1 a includes a plurality of annular elements 10 aligned longitudinally adjacent to each other along a longitudinal axis 15. Although only one annular element need be provided, it is preferable that the endoprosthesis includes a plurality of annular elements 10, depicted herein for the purpose of illustration by at least a first annular element 10a and a second annular element 10b.

Each annular element 10 includes a set of interconnected strut elements shown as strut crossbars 20, which are disposed circumferentially about the longitudinal axis 15. Arrows 17 illustrates the circumferential directionality. Each crossbar 20 has a first end 22a and a second end 22b, referenced generally as end 22. The second end 22b of selected circumferentially-adjacent crossbars 20a-b are interconnected at elbows 30 that are proximate to a first longitudinal side 12 of each annular element 10, and the first end 22a of selected circumferentially adjacent crossbars 20b-c are interconnected to define elbows 30 that are proximate to a second longitudinal side 14 of the annular element.

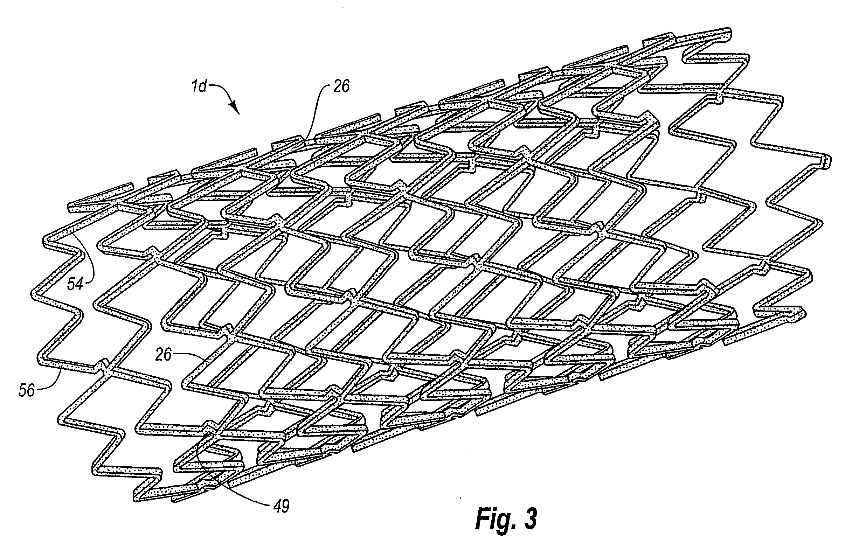

Each annular element 10 can be expanded to a deployed configuration as shown in Figure 3 by altering or opening the angle of the elbows 30 interconnecting the circumferentially-adjacent crossbars 20. Also, circumferentially-adjacent elbows 30 on each side 12, 14 of the annular element 10 can be spaced apart by a circumferential distance D, such that each annular element 10 is expanded by increasing the distance D between circumferentially-adjacent elbows 30. At any given condition between the delivery configuration and the deployed configuration, the distance D can be balanced or constant from one set of circumferentially-adjacent elbows to the next, or can be varied if desired.

Selected elbows 30 on each side 12, 14 of the annular element 10 are defined by interconnecting corresponding ends 22 of circumferentially-adjacent crossbars 20a-b directly together to form a zigzag pattern of alternating U-shapes, V-shapes, L-shapes, combinations thereof, or the like when deployed. Alternatively, an elbow 30 can be provided between the corresponding ends of adjacent crossbars to form another contoured shape, such as by using a straight elbow member to form a flat connection or a curved elbow member to form an arcuate connection configuration.

Figure 1 also depicts an example of a foot extension 40 that extends between a pair 24 of circumferentially-adjacent crossbars 20d-e of each annular element 10. As depicted, the foot extension 40 includes an ankle 41 that circumferentially couples an end 22 of one of the adjacent crossbars 20d to a medial segment 44. The medial segment 44 extends from the ankle 41 to a toe 48 that circumferentially couples the medial segment to a lateral segment 46. The lateral segment 46 extends from the toe 48 to a heel 42 that circumferentially couples the lateral segment to the next circumferentially-adjacent crossbar 20e. Accordingly, the juncture of the crossbar 20d and the medial segment 44 defines a circumferentially extending toe portion 48 of the foot extension 40; the juncture of the medial segment 44 and the lateral segment 46 defines a circumferentially-extending toe portion 48 of the foot extension 40; and the juncture of the lateral segment 46 and crossbar 20e defines a circumferentially-extending toe portion 48 of the foot extension 40.

Additionally, for the purpose of discussion and not limitation, Figure 1 shows that a toe portion 48 can extend in a first circumferential direction at a distance greater than the heel portion 42 of the foot extension 40 extends in an opposite circumferential direction. As such, the entirety of the foot extension 40 can extend in the circumferential direction of the toe portion 48. Furthermore, at least one of the medial segment 44 or lateral segment 46 can open to form an open foot region 49 (see also, Fig. 3). Additionally, a variety of other configurations can be used for the foot extension.

For example, the foot extension generally extends from the pair of circumferentially-adjacent strut members circumferentially at an angle relative to a line parallel to the longitudinal axis of the annular element. Figure 1 shows a foot extension 40 generally extending circumferentially at an angle of about 90 degrees relative to the longitudinal axis 15. However, the foot extension can be configured to extend circumferentially at any angle relative to the longitudinal axis.

In one example of a foot extension 40, the medial segment 44 and the lateral segment 46 can be generally parallel, straight elongate portions joined by a curved toe portion 48. Particularly, the lateral segment 46 can be defined by a generally straight portion and each of the toe portion 48, the ankle portion 41, and the heel portion 42 can be defined by a curved portion. Each portion of the foot extension 40, as well as each of the circumferentially-adjacent crossbars 20, has a substantially uniform cross-sectional profile illustrated by a substantially uniform width W and thickness (not shown). Alternatively, the strut elements can have varying or different widths and/or thicknesses. The foot extension 40 can thus generally define at least two areas of flexure between the pair of circumferentially-adjacent strut members 20d-e. That is, one at the heel portion 42 and one at the ankle portion 44. An additional or alternative area of flexure can be defined at the toe portion 48 if desired to facilitate further expansion between the pair of circumferentially-adjacent crossbars 20, such as to define a three-point hinge configuration.

In the illustrated endoprosthesis 1a, a plurality of connectors 60 are be provided to connect adjacent annular elements 10c-d at a plurality of connection locations 50. Each connection location 50 can include a foot extension 40 of one annular element 10d and an elbow 30 of the adjacent annular element 10c, with a connector 60 having opposite ends 62a-b connected therebetween. Alternatively, the connector 60 can extend from elbow-to-elbow, foot-to-foot, foot-to-elbow, crossbar-to-crossbar, combinations thereof, and the like.

As shown, the adjacent annular elements 10c-10d can be coupled by an elbow connector end 62a being coupled to an elbow 30, and an opposite foot connector end 62b being coupled to a foot 40. More particularly, the foot connector end 62b of the connector 60 can be coupled to the lateral segment 46 of the foot extension 40. With a connector 60 extending longitudinally from the lateral segment 46 of a foot extension 40 to an elbow 30, the longitudinally-adjacent elbows 30 of adjacent annular elements 10c-10d can be circumferentially out of alignment. The foot 40 lateral segments 46 at the longitudinal ends 16, 18 of the endoprosthesis 1 a face outward from the remainder of the structure.

Preferably, one or more foot extensions 40 at either end 16, 18 of the endoprosthesis 1 a can include an area that undergoes minimal deformation or strain, such as the lateral segment 46, when expanded to the deployed configuration. A wire or strip of radiopaque material can be wrapped around or otherwise secured to this area of minimal strain so as to act as a radiopaque marker 28 for imaging purposes. Also, the radiopaque marker material can be used to fill the holes within lateral segment 46, or within the holes of any strut element described herein.

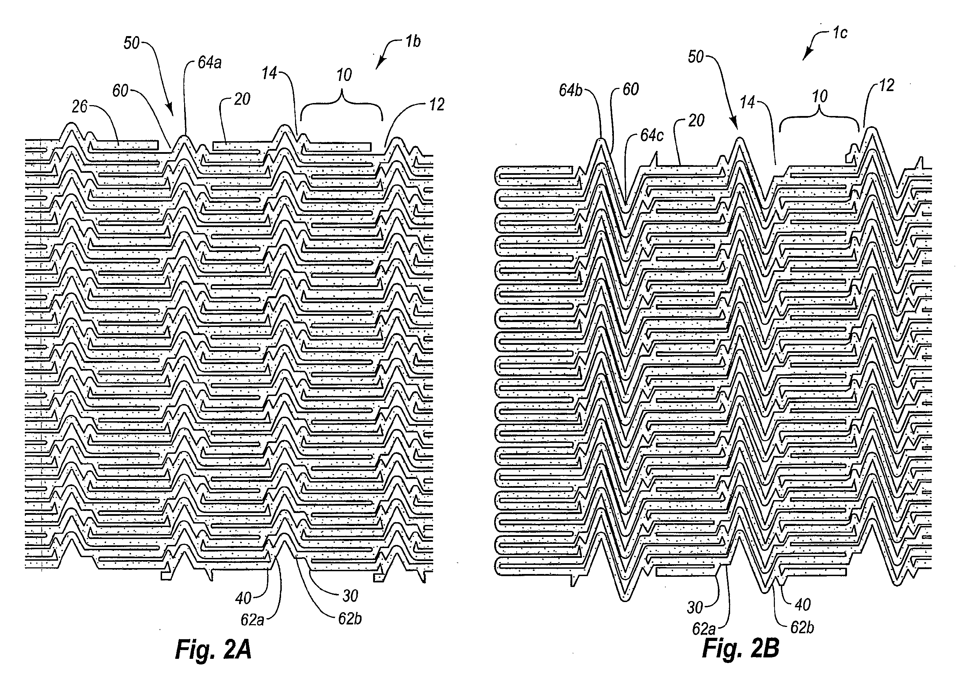

For simplicity and clarity, each crossbar 20 and connector 60 depicted in Figure 1 is shown to be a straight member. It is recognized, however, that the crossbars 20 and connectors 60 can be contoured or shaped to increase longitudinal flexibility if desired, as shown in Fig 2A-2B. As shown, the embodiments of the endoprosthesis 1b-1c shown in Figures 2A-2B, respectively, can include the various strut elements and holes 26 depicted and described in connection with Figure 1. Additionally, Figure 2A shows a connector 60 having one apex 64a, and Figure 2B shows a connector have two apexes 64b-64c. Similarly, the connectors 60 need not extend parallel to the longitudinal axis 15, but can be aligned diagonally or helically such that the ends of the connector are circumferentially offset.

Additionally, each crossbar 20 of the annular element 10 can be a straight member or have various curves or shapes similar to the connectors 60 shown in Figures 2A-2B. Preferably, when in a closed delivery configuration, the crossbars 20 are generally aligned to be substantially parallel with the longitudinal axis 15, as well as with each other as shown by the embodiments of the endoprostheses 1 b-1 c in Figures 2A-2B. Additionally, the crossbars 20 of each annular element 10 can be interconnected with circumferentially-adjacent crossbars 20 to form a continuous closed ring, such as depicted more clearly by the embodiment of the deployed tubular endoprosthesis 1d in Figure 3. Accordingly, the tubular endoprosthesis 1d is shown in a deployed and expanded orientation. The tubular endoprosthesis 1d is illustrated to show the sides 54 and tops 56, both of which can include the plurality of holes 26.

Although not specifically shown, alternative shapes can be used in addition to or in lieu of the straight crossbars and/or connectors, such as L-shaped, U-shaped or V-shaped crossbars and/or connectors or the like as is known in the art. The elbows, feet, ankles, toes, foot medial segments, foot lateral segments, connectors, and the like can also have shapes other than those which are illustrated. The number of crossbars or other strut elements included in each annular element can depend upon the size and desired characteristics of the endoprosthesis. For example, a greater number of crossbars, elbows, feet, and/or other strut elements can be provided for increased surface-area coverage of the luminal wall by the endoprosthesis or increased cross-sectional profile of the endoprosthesis in the deployed configuration.

Similarly, the radial bias, rigidity, flexibility, crack resistance, fatigue resistance, and like physical characteristics of each annular element can be controlled or varied by altering the shape or size of the crossbars, elbows, feet, connectors, and the like. The physical characteristics of an annular element, when deployed, generally can be increased by decreasing the length or by modifying the cross-sectional profile of selected strut elements of the annular element. For example, it is possible to provide an endoprosthesis having varied radial bias or rigidity along its length by providing one annular element with a radial bias or rigidity that is different from the radial bias or rigidity of another annular element as is well known in the art. In a similar manner, it is possible to provide an endoprosthesis having a tapered or flared shape formed of adjacent annular elements having different cross-sectional profiles when in the deployed configuration, but having a similar or uniform radial bias or rigidity along its length.

### II. Endoprosthetic Elements

The improvement in endoprosthetic elements can improve the overall structural integrity of an endoprosthesis. The improved endoprosthetic elements include various types of holes in an amount and arrangement that can inhibit crack formation and/or propagation and/or fatigue-induced catastrophic failure, thereby improving the performance and reliability of the endoprosthesis. For example, any particular endoprosthetic element can include rounded walls therein that define a hole, wherein it is well known that rounded features are less susceptible to crack formation compared to sharp features. Additionally, the positioning of holes within the body of a element can serve to inhibit crack propagation because when a crack encounters a hole the crack terminates and may not extend therethrough. Also, the hole at a terminal end of a crack can allow for the body to flex so that it is less stressed.

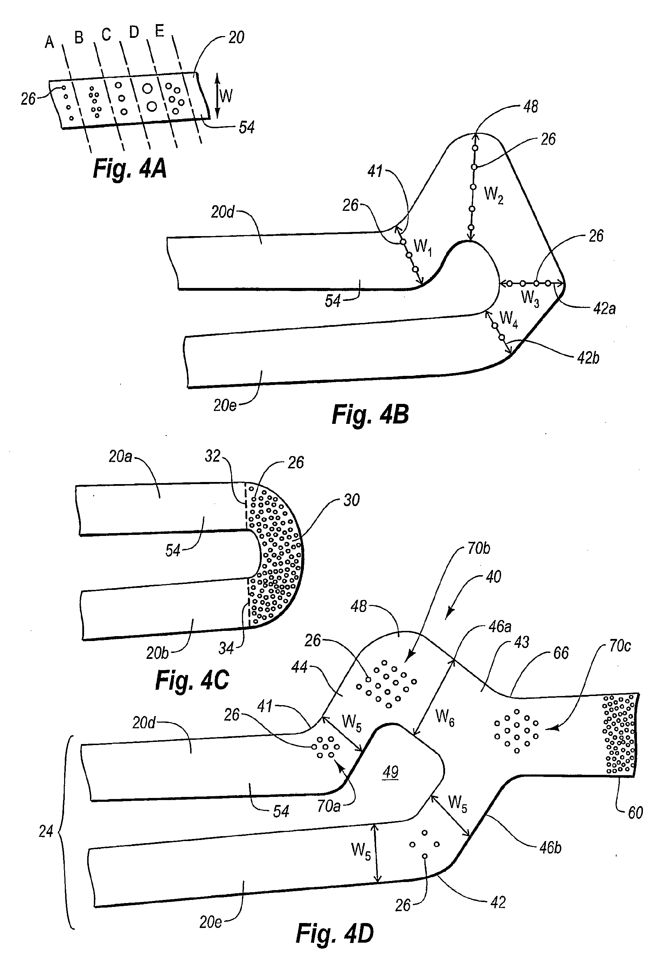

Figure 4A depicts a crossbar 20 substantially similar to that - of Figure 1. The crossbar 20 is illustrated to include sections A-B for explanatory purposes and not for limitation. It will be understood that crossbar 20 and any other portion of the endoprosthesis, - include a plurality of holes, the plurality of holes being arranged in accordance with one or more of the Sections A-E. As shown, each of the sections A-E includes a plurality of holes 26 distributed across the width W of the crossbar 20 in order to provide improved structural integrity by inhibiting crack formation and/or crack propagation and/or fatigue-induced catastrophic failure. Sections A-E have the following configurations: section A illustrates a plurality of holes (e.g., 4 holes) aligned along the width W; section B illustrates a pattern of holes distributed along the Width in a 1-2-1-2-1 arrangement; section C illustrates 3 holes aligned along the width W; section D illustrates 2 holes aligned along the width W; and section E illustrates 5 holes in a zigzag pattern along the width W. While sections A-E illustrate holes in various numbers and arrangements across a given width W, various other numbers and arrangements that provide improved structural integrity can also be used. In still other configurations, the arrangement of the holes can be random, while still providing improved structural integrity. Moreover, these and other hole numbers and arrangements can be used for other strut elements such as connectors, elbows, foot extensions, ankles, toes, heels, medial segments, lateral segments, and the like. More examples of hole amounts and arrangements are described in more detail below.

Figure 4B depicts a foot extension 40 substantially similar to that of Figure 1. The foot extension 40 is shown to be disposed between and coupling a first crossbar 20d and a second crossbar 20e While the first and second crossbars 20d-e are shown to be substantially devoid of holes, it should be recognized that any type of hole in any amount or number and/or distribution can be present, wherein the holes are omitted for clarity. The foot extension 40 is shown to have various flexible portions shown as the ankle 41, toe 48, and heel bend 42a-b. The flexible portions can be stressed during deployment and after being deployed, which can result in such flexible portions being susceptible to crack formation and/or crack propagation and/or fatigue-induced catastrophic failure. In order to inhibit such unfavorable consequences; the flexible portions include holes in amounts and arrangements that provide improved structural integrity. The ankle 41 is depicted to include a width W1 that has 3 holes 26 that are aligned linearly. The toe 48 is depicted to include a width W2 that has 5 holes 26 that are aligned linearly. The first ankle bend 42a is depicted to include a width W3 that has 4 holes 26 that are aligned linearly. The second ankle bend 42b is depicted to include a width W4 that has 2 holes 26 that are aligned linearly. While various amounts of holes are shown to be linearly aligned, the holes can be in any of various other amounts or arrangements. For example, the holes can be in patterns around the entire foot extension 40 or in patterns at discrete locations or locations more susceptible to stress.

Figure 4C depicts an elbow 30 substantially similar to that of Figure 1. The elbow 30 is shown to be disposed between and coupling a first crossbar 20a and a second crossbar 20b. While the first and second crossbars 20a-b are shown to be substantially devoid of holes, it should be recognized that any type of hole in any amount and/or distribution can be present, wherein the holes are omitted for clarity. The elbow 30 is shown to be a flexible junction between the crossbars 20a-b, and thereby is subjected to stresses during deployment and after being deployed, which can result in the elbow being susceptible to crack formation and/or crack propagation and/or fatigue-induced catastrophic failure. In order to inhibit such unfavorable consequences; the elbow 30 includes holes in amounts and arrangements that provide improved structural integrity. Accordingly, the elbow is shown to have a plurality of holes 26 in a random arrangement that extends from a first end 32 to a second end 34 of the elbow 30.

Figure 4D depicts a foot extension 40 substantially similar to that of Figure 1 and Figure 4B. Additionally, the foot extension 40 is coupled to a connector 60 that couples one annular element to another annular element. The foot extension 40 is shown to be disposed between and coupling a first crossbar 20d and a second crossbar 20e, which are both coupled to the connector 60. While the first and second crossbars 20d-e are shown to be substantially devoid of holes, it should be recognized that any type of hole in any amount and/or distribution can be present, wherein the holes are omitted for clarity. The foot extension 40 is shown to have various flexible portions shown as the ankle 41, toe 48, heel 42, and connector junction 66. Similar to Figure 4B, the connector junction 66 can also be stressed during deployment and after being deployed, which can result in such flexible portions being susceptible to crack formation and/or crack propagation and/or fatigue-induced catastrophic failure. In order to inhibit such unfavorable consequences; the flexible portions include holes in amounts and arrangements that provide improved structural integrity. The ankle 41 is depicted to include a pattern 70a that has 7 holes 26 that have an alternating arrangement. The toe 48 is depicted to include a pattern 70b that has 16 holes 26 that are in an aligned arrangement. The ankle 41 is depicted to include a pattern 70c that has 4 holes 26 that form a square arrangement. The connector junction 66 is depicted to include a pattern 70d with 14 holes 26 that have an alternating arrangement. While various amounts and patterns of holes are shown to be disposed at locations susceptible to stress, the holes can be in any amount and arrangement and can also be disposed at the medial segment 44, first lateral segment 46a, and second lateral segment 46b.

Additionally, the toe 48, first lateral segment 46a, and connector junction 66 are shown to have wider widths W6 compared to the narrower widths W5 of the ankle 41, second lateral segment 46b, and heel 42. The wider widths W6 can provide more area so that a larger number of holes 26 can be used compared to the narrower widths W5. Accordingly, the width of a stress plane and the portion of the surrounding strut element can be increased to have a wider width W6 to accommodate more holes. Inversely, the areas of the strut elements that are not as susceptible to stress can have narrower widths W5 and may need fewer holes. In any event, the width of a strut element can be modulated with the amount and arrangement of holes in order to increase structural integrity and inhibit crack formation and/or crack propagation and/or fatigue-induced catastrophic failures.

It will be appreciated, and with reference to Figure 3, that the holes 26 can be disposed through the sides and/or tops of the strut elements. More generally, the holes 26 can be disposed through any portion of the strut elements.

### III. Features

An endoprosthesis in accordance with the present invention includes holes in order to improve structural integrity and to inhibit crack formation and/or crack propagation and/or fatigue-induced catastrophic failure. The foregoing discussion of the endoprosthesis described the holes to be located in any of the prosthetic elements. It will be understood that other features can be used and the following discussion regarding the inclusion of holes in no way limits the use of other additional features. Briefly, various amounts of holes randomly arranged were described to be implemented at different locations of the endoprosthetic elements, especially at stress planes where an element may be more susceptible to crack formation and/or crack propagation. Accordingly, the various hole configurations at a particular stress plane or at any other location are described in more detail below.

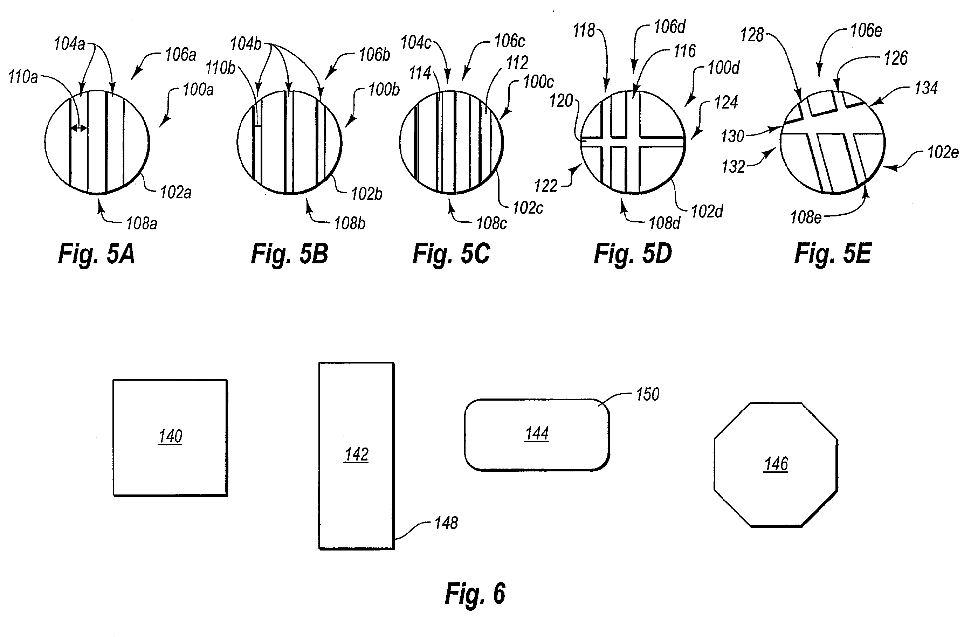

Figure 5A is an example of a cross-sectional profile 100a of a prosthetic element 102 that includes a plurality of holes 104a. Accordingly, two holes 104a can be positioned within a cross-sectional plane of the element 102a. As shown, the holes 104a extend from a first side 106a to a second side 108a. The holes 104a are shown to be evenly spaced within the interior of the element 102a, and each has a substantially uniform diameter 110. Moreover, the holes 104 are shown to have a diameter 110a.

Figure 5B is another example of a cross-sectional profile 100b of a prosthetic element 102b that includes a plurality of holes 104b. Accordingly, three holes 104b can be positioned within a cross-sectional plane of the element 102b. As shown, the holes 104b extend from a first side 106b to a second side 108b. The holes 104b are shown to be evenly spaced within the interior of the element 102b, and each has a substantially uniform diameter 110b. Moreover, the holes 104b are shown to have a diameter 110b narrower than that of the diameter 110 (Figure 5A).

Figure 5C is another example of a cross-sectional profile 100c of a prosthetic element 102c that includes a plurality of holes 104c. Accordingly, four holes 104c can be positioned within a cross-sectional plane of the element 102c. As shown, the holes 104c extend from a first side 106c to a second side 108c. The holes 104c are shown to be unevenly spaced within the interior of the element 102b. Also, the outer holes 112 are shown to be narrower compared to the inner holes 114.

Figure 5D is another example of a cross-sectional profile 100d of a prosthetic element 102d that includes a plurality of holes 116, 118, 120. Accordingly, three holes 116, 118, 120 can be positioned within a cross-sectional plane of the element 102d. As shown, two holes 116, 118 extend from a first side 106d to a second side 108d, and are asymmetrically disposed with the first hole 116 at the center and the second hole 118 closer to a medial side 122. The third hole 120 extends from the medial side 122 to the lateral side 124.

Figure 5E is another example of a cross-sectional profile 100e of a prosthetic element 102e that includes a plurality of holes 126, 128, 130. Accordingly, three holes 126, 128, 130 can be positioned within a plane of the element 102e. As shown, two holes 126, 128 extend from a first side 106e to a second side 108e, and are disposed randomly. The third hole 130 extends from the medial side 132 to the lateral side 134, and forms an intersection with the first hole 126 and the second hole 128. As shown, the third hole 130 is slightly out of the cross-sectional plane so that the opening at the medial side 132 appears smaller than the opening at the lateral side 134 so that it extends from behind the plane at the medial side 132 to in front of the plane at the lateral side 134.

Figure 6 illustrates various other examples of cross-sectional profiles of a prosthetic element which are different from the circular, curved, elliptical, or ovular configurations of Figures 5A-5E. The cross-sectional profiles are illustrated without holes for simplicity; however, they can have any possible amount or arrangement of holes. As such, the cross-sectional profile of the prosthetic element can optionally be square 140, orthogonally rectangular 142, circumferentially rectangular 144, octagonal 146, combinations thereof, or the like. Also, the cross-sectional profile can have sharp corners 148, rounded corners 150, or combinations thereof.

Figures 5A through 6 illustrate exemplary cross-sectional profiles of strut members of an endoprosthesis. In Figures 5A through 5E the endoprosthesis is shown having a circular cross-sectional profile while in Figure 6 the endoprosthesis is shown having other geometric cross-sectional profiles. It shall be understood that the cross-sectional profiles shown are merely exemplary and that the cross-sectional profile of the endoprosthesis may be any shape. As known to one of ordinary skill in the art the cross-sectional profile of an endoprosthesis can be shaped by cutting, electro-polishing, grinding, etching and other known processes to produce any desired shape.

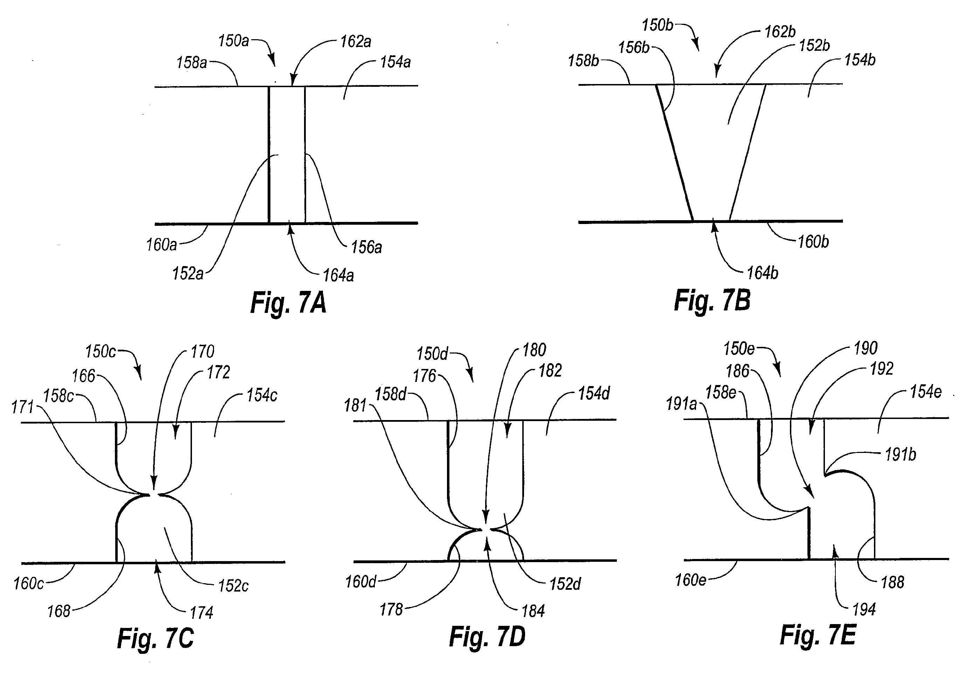

Figure 7A illustrates a cross-sectional profile 150a of a hole 152a disposed within an endoprosthetic element 154a. The hole 152a is shown to be defined by a round wall 156a that extends from the first surface 158a to a second surface 160a. Accordingly, the hole 152a has a first opening 162a and second opening 164a that have substantially the same diameter so as to form a hole having a substantially uniform diameter from the first surface 158a to the second surface 160a. A hole 152a having a substantially uniform diameter can be prepared by drilling through the material of the endoprosthetic element, wherein such drilling is described in more detail below.

Figure 7B illustrates another cross-sectional profile 150b of a hole 152b disposed within an endoprosthetic element 154b. The hole 152b is shown to be defined by a round wall 156b, although other shapes are possible, that extends from the first surface 158b to a second surface 160b. Accordingly, the hole 152b has a first opening 162b and second opening 164b that have substantially different diameters, wherein the first opening 162b has a diameter that is substantially larger than the second opening 164b. Thus, the hole 152b has a tapered diameter that decreases from the first surface 158b to the second surface 160b or vice versa. A hole 152b having a tapered diameter can be prepared by forming a hole through the material of the endoprosthetic element in a manner that may require less precision than holes with uniform diameters. Usually, the narrowing taper is less than about 15 degrees, more preferably less than about 10 degrees, and most preferably less than about 5 degrees.

Figure 7C illustrates yet another cross-sectional profile 150c of a hole 152c disposed within an endoprosthetic element 154c. The hole 152c is shown to be defined by a first recess wall 166 that extends from the first surface 158c to a midpoint 171, and by a second recess wall 168 that extends from the midpoint 171 to the second surface 160c. Accordingly, the hole 152c has a first recess 172 and a second recess 174 that are fluidly coupled by an opening 170 at the midpoint 171. The first recess 172 and second recess 174 are substantially similar in shape and depth. The fluid coupling of the recesses 172, 174 at the opening 170 allows for partial holes or recesses that interconnect to be formed in each side 158c, 160c of the endoprosthetic element 154c.

Figure 7D illustrates yet another cross-sectional profile 150d of a hole 152d disposed within an endoprosthetic element 154d. The hole 152d is shown to be defined by a first recess wall 176 that extends from the first surface 158d to an asymmetrical point 181, and by a second recess wall 178 that extends from the asymmetrical point 181 to the second surface 160d. Accordingly, the hole 152d has a first recess 182 and a second recess 184 that are fluidly coupled by an opening 180 at the asymmetrical point 181. As such, the first recess 182 has a larger size and depth compared to the second recess 184. The fluid coupling of the recesses 182, 184 at the opening 180 allows for partial holes to be formed in each side 158d, 160d of the endoprosthetic element 154d, wherein the first recess 182 is burrowed further than the second recess 184.

Figure 7E illustrates yet another cross-sectional profile 150e of a hole 152e disposed within an endoprosthetic element 154e. The hole 152e is shown to be defined by a first recess wall 186 that extends from the first surface 158e to an offset plane 191 a-191 b, and by a second recess wall 188 that extends from the offset plane 191 a-191 b to the second surface 160e. Accordingly, the hole 152e has a first recess 192 and a second recess 194 that are fluidly coupled by an opening 190 at the offset plane 191a-191b. As such, the first recess 192 has an offset fluid coupling with the second recess 194. The fluid coupling of the recesses 192, 194 at the opening 190 allows for partial holes that are offset to be formed in each side 158e, 160e of the endoprosthetic element 154e.

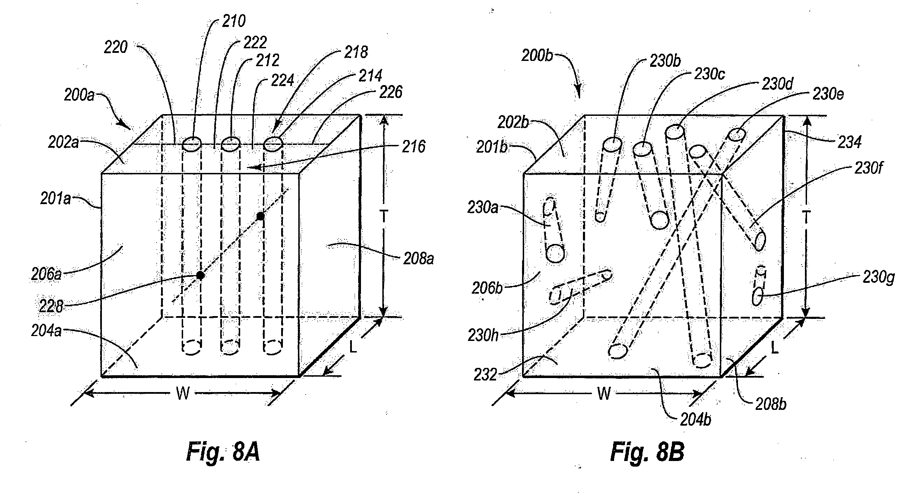

Figure 8A illustrates a selected volume of an endoprosthetic element 200a having a substantially square cross-sectional profile. The element 200a is defined by a top side 202a opposite a bottom side 204a and an inner side 206a opposite an outer side 208a. Accordingly, the element body 201 a between the top side 202a and the bottom side 204a defines the thickness of the element 200a, and the element body between the inner side 206a and the outer side 208a defines the width of the element. The element 200a is depicted to include a first hole 210, second hole 212, and third hole 214, wherein each hole extends from the top side 202a through the thickness of the body 201 a to the bottom side 204a. Also, the holes 210, 212, 214 are linearly aligned across the width. As such, a first portion 216 of the body 201 a can be coupled to a second portion 218 of the body 201 a by the following: a lateral inner connecting member 220 that is disposed between the inner side 206a and the first hole 210; a medial inner connecting member 222 that is disposed between the first hole 201 and the second hole 212; a medial outer connecting member 224 that is disposed between the second hole 212 and the third hole 214; and a lateral outer connecting
member 226 that is disposed between the third hole 214 and the outer side 208a. While the element 200a is defined by a top side 202a, opposite a bottom side 204a and an inner side 206a opposite an outer side 208a, it should be recognized such sides could be swapped by rotating the body 201 a about a longitudinal axis 228 along the length of the element.

Figure 8B illustrates another selected volume of an endoprosthetic element 200b having a substantially square profile. The element 200b is defined by a top side 202b opposite a bottom side 204b and an inner side 206b opposite an outer side 208b. Accordingly, the element body 201b between the top side 202b and the bottom side 204b defines the thickness of the element 200b, and the element body between the inner side 206b and the outer side 208b defines the width of the element. The element 200b is depicted to include random holes 230a-h having random orientations with respect to the body 201 b. For clarity a proximal surface 232 and a distal surface 234 are shown on the body 201 b in order to illustrate the longitudinal aspect of the trajectory of some of the holes 230. As such, any hole 230 that intersects the proximal surface 232 or distal surface is assumed to continue its trajectory until intersecting with one of the top-side 202b, bottom side 204b, inner side 206b, or outer side 208b.

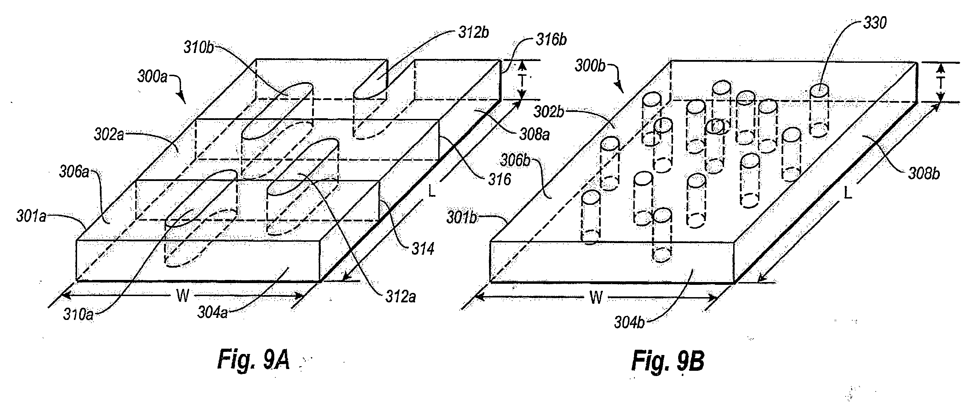

Figure 9A illustrates another selected volume of an endoprosthetic element 300a having a substantially rectangular cross-sectional profile. The element 300a is defined by a top-side 302a opposite a bottom side 304a and an inner side 306a opposite an outer side 308a. Accordingly, the element body 301 a between the top-side 302a and the bottom side 304a defines the thickness of the element 300a, and the element body between the inner side 306a and the outer side 308a defines the width of the element. The element 300a is depicted to include a first inner hole 310a and second inner hole 310b linearly aligned along the length, and a first outer hole 312a and second outer hole 312b linearly aligned along the length. Additionally, the first inner hole 310a and first outer hole 312a are staggered with respect to the width, and the second inner hole 310b and second outer hole 312b are also staggered with respect to the width. The staggered configuration allows for some cross-sectional planes 314 to intersect two holes (e.g., 310a, 312a) and some cross-sectional places 316 to intersect only one hole (e.g., 310b).

Figure 9B illustrates another selected volume of an endoprosthetic element 300b having a substantially rectangular cross-sectional
profile. The element 300b is defined by a top-side 302b opposite a bottom side 304b and an inner side 306b opposite an outer side 308b. Accordingly, the element body 301 b between the top-side 302b and the bottom side 304b defines the thickness of the element 300b, and the element body between the inner side 306b and the outer side 308b defines the width of the element. The element 300b includes a plurality of holes 330 arranged in a random distribution. Generally, each of the holes 330 extends from the top-side 302b through the body 301 b to the bottom side 304b.

The size of the holes or the cumulative size of the holes across a cross-sectional profile of an endoprosthetic element can be modulated to correspond with the width, thickness, and/or cross-sectional profile of the endoprosthetic element. For example, stents having crossbars with widths and/or thicknesses of from about 55 micron to about 250 micron can include a plurality of holes across a given width, thickness and/or cross-sectional profile. As such, the size of the individual holes or the summation of the size of all the holes across a given width, thickness, and/or cross-sectional profile can be tailored to provide sufficient structural integrity.

In one embodiment, an endoprosthetic element has a width, thickness, or cross-sectional profile that includes at least two holes. The two holes across the endoprosthetic element each have a diameter or width that partition the element into at least a first portion and a section portion. The first portion and second portion are connected by at least three connection elements. Each of the three connection elements can have a sufficient strength so that only two of the connection elements are required to provide structural integrity.

Additionally, the diameter or width of each hole that is situated at a given width, thickness, and/or cross-sectional profile can be varied to correspond with size limitations of the endoprosthetic element.

As recognized from the detailed description above, the holes enhance structural integrity and provide versatility in the design of the endoprosthesis of the present invention. The holes can be configured and dimensioned relative to the endoprosthetic elements to improve structural integrity. For example, the amount and distribution of holes at areas of flexure of the elbow, foot extensions, and junctions can be adjusted depending on stress, susceptibility of cracking, and the like. Alternatively, the size and/or geometry of the holes can be configured to correspond with the size and/or geometry of the endoprosthetic element. The holes can be configured to balance or assist in evenly distributing strain or expansion of the endoprosthesis. The holes also can improve and control the flexibility of the endoprosthesis, preferably without substantially impacting the desired coverage or scaffolding of the endoprosthesis.

### IV. Endoprosthetic Composition

The endoprostheses of the present invention can be made of a variety of materials, which are well known in the art of endoprosthesis manufacturing. The material of construction can be selected according to the structural performance and biological characteristics that are desired. For example, an endoprosthesis of the present invention can be made to be expanded by the change of a delivery condition, such as by the removal of a restraint or exposure to the environment within the body lumen so as to be self expanding, or by the application of an external force or energy, such as by a balloon or by a radio frequency. For purpose of illustration and not limitation, reference is made generally to "self-expanding" embodiments and "balloon expandable" embodiments of the endoprosthesis of the present invention.

Self-expanding embodiments of an endoprosthesis can be made from any of a variety of known suitable materials, such as a shaped memory material ("SMM"). For example, the SMM can be shaped in a manner that allows for restriction to induce a substantially tubular, linear orientation while within a delivery shaft, but can automatically retain the memory shape of the endoprosthesis once extended from the delivery shaft. SMMs have a shape memory effect in which they can be made to remember a particular shape. Once a shape has been remembered, the SMM may be bent out of shape or deformed and then returned to its original shape by unloading from strain or by heating. Typically, SMMs can be shape memory alloys ("SMA") comprised of metal alloys, or shape memory plastics ("SMP") comprised of polymers.

Usually, an SMA can have any non-characteristic initial shape that can then be configured into a memory shape by heating the SMA and conforming the SMA into the desired memory shape. After the SMA is cooled, the desired memory shape can be retained. This allows for the SMA to be bent, straightened compacted, and placed into various contortions by the application of requisite forces; however, after the forces are released the SMA is capable of returning to the memory shape. The main types of SMAs are as follows: copper-zinc-aluminium; copper-aluminium-nickel; nickel-titanium ("NiTi) alloys known as nitinol; and cobalt chromium-nickel alloys or cobalt-chromium-nickel-molybdenum alloys known as elgiloy. However, other types of SMAs can be used. Typically, the nitinol and elgiloy alloys are more biocompatible, and have superior mechanical characteristics in comparison with the copper-based SMAs. The temperatures at which the SMA changes its crystallographic structure are characteristic of the alloy, and can be tuned by varying the elemental ratios.

For example, it can be preferable for the primary material of an endoprosthesis to be comprised of a Ni-Ti alloy that forms superelastic nitinol. In the present case, nitinol materials can be trained to remember a certain shape, straightened in a shaft, catheter, or other tube, and then released from the catheter or tube to return to its trained shape. Also, additional materials can be added to the nitinol depending on the desired characteristic.

An SMP is a shape-shifting plastic that can be fashioned into an endoprosthesis in accordance with the present invention. When an SMP encounters a temperature above the lowest melting point of the individual polymers, the blend can make a transition to a rubbery state. The elastic modulus can change more than two orders of magnitude across the transition temperature ("Ttr"). As such, an SMP can be formed into a desired shape of an endoprosthesis by heating it above the Ttr, fixing the SMP into the new shape, and cooling the material below the Ttr. The SMP can then be arranged into a temporary shape by force, and then resume the memory shape once the force has been applied. Examples of SMPs include biodegradable polymers, such as oligo(ε-caprolactone)diol, oligo(p-dioxanone)diol, and non-biodegradable polymers such as, polynorborene, polyisoprene, styrene butadiene, polyurethane-based materials, vinyl acetate-polyester-based compounds, and others yet to be determined. As such, any SMP can be used.

For example, Verifiex™, the trade name for CRG's family of shape memory polymer resin systems, currently functions on thermal activation which can be customizable from -28.9°C to 271.1°C (20 °F to 520° F), which allows for customization within the range of normal body temperature. This allows an endoprosthesis comprised of Veriflex™ to be inserted into a delivery catheter. Once unrestrained by the delivery shaft, the body temperature can cause the endoprosthesis to spontaneously take its functional shape.

An endoprosthesis made of a SMM or suitable superelastic material can be compressed or restrained in its delivery configuration on a delivery device using a sheath or similar restraint, and then deployed to its deployed configuration at a desired location by removal of the restraint as is known in the art. An endoprosthesis made of a thermally sensitive material can be deployed by exposure of the endoprosthesis to a sufficient temperature to facilitate expansion as is known in the art.

Balloon expandable endoprostheses embodiments can be made of any of a variety of known suitable deformable materials, including stainless steel, silver, platinum, cobalt-chromium alloys or other known biocompatible materials.

For delivery, the balloon-expandable endoprosthesis of a suitable material can be mounted in the delivery configuration on a balloon or similar expandable member of a delivery device. Once properly positioned within the body lumen at a desired location, the expandable member, such as a balloon, can be expanded to expand the endoprosthesis to its deployed configuration as is known in the art.

Also, balloon expandable endoprostheses embodiments can be made of suitable biocompatible polymers in addition to or in place of a suitable metal or alloy. The polymeric endoprostheses can include biodegradable or bioabsorbable materials, which can be either plastically deformable or capable of being set in the deployed configuration. If plastically deformable, the material can be selected to allow the endoprosthesis to be expanded in a similar manner using an expandable member so as to have sufficient radial strength and scaffolding and also to minimize recoil once expanded. If the polymer must be set in the deployed configuration, the expandable member can be provided with a heat source or infusion ports to provide the required catalyst to set or cure the polymer. Alternatively, known delivery devices and techniques for a self-expanding endoprosthesis can be used.

Additionally, an embodiment of an endoprosthesis can be comprised of a biocompatible material capable of expansion upon exposure to the environment within the body lumen, or other well-known means for expansion. Examples of such biocompatible materials can include a suitable hydrogel, hydrophilic polymer, biodegradable polymers, bioabsorbable polymers. Examples of such polymers can include poly(alpha-hydroxy esters), polylactic acids, polylactides, poly-L-lactide, poly-DL-lactide, poly-L-lactide-co-DL-lactide, polyglycolic acids, polyglycolide, polylactic-co-glycolic acids, polyglycolide-co-lactide, polyglycolide-co-DL-lactide, polyglycolide-co-L-lactide, polyanhydrides, polyanhydride-co-imides, polyesters, polyorthoesters, polycaprolactones, polyesters, polyanydrides, polyphosphazenes, polyester amides, polyester urethanes, polycarbonates, plytrimethylene carbonates, polyglycolide-co-trimethylene carbonates, poly(PBA-carbonates), polyfumarates, polypropylene fumarate, poly(p-dioxanone), polyhydroxyalkanoates, polyamino acids, poly-L-tyrosines, poly(beta-hydroxybutyrate), polyhydroxybutyrate-hydroxyvaleric acids, combinations thereof, or the like. For example, a self-expandable endoprosthesis can be delivered to the desired location in an isolated state, and then exposed to the aqueous environment of the body lumen to facilitate expansion.

Additionally, other well-known delivery devices and techniques for a self-expanding endoprosthesis can be used. For example, prior to crimping of the self-expanding endoprosthesis for loading into a delivery system, the endoprosthesis may be coated with a lubricant such as silicone oil to reduce the force between the endoprosthesis and the crimping device and additionally to reduce the forces associated with disposing the endoprosthesis in a delivery device. Additionally, the lubricant may reduce the deployment force thereby increasing the accuracy of endoprosthesis placement within a patient. The lubricant may be introduced prior to, during, or after the crimping or loading process.

### A. Filled Features

It is further contemplated that the holes in the prosthetic elements can be filled with another material having a composition different from the primary endoprosthetic material. The use of a different material to fill the holes can be beneficial for imparting additional properties to the endoprosthesis, such as providing radiopaque characteristics, drug-reservoirs, and structural reinforcement.

In one embodiment, the holes of an endoprosthesis can be filled with a biocompatible material. For example, the endoprosthesis can include holes in the primary material or matrix that are filled or coated with one or more coatings of a biocompatible material to enhance the biocompatibility of the device. Such fillings and/or coatings can include hydrogels, hydrophilic and/or hydrophobic compounds, polypeptides, proteins, amino acids, combinations thereof, and/or the like. Specific examples can include polyethylene glycols, polyvinylpyrrolidone ("PVP"), polyvinylalcohol ("PVA"), parylene, heparin, and the like. A preferred filling and/or coating material can include phosphorylcholine, as disclosed in U.S. Patent No. 6,015,815 entitled "TETRAZOL-CONTAINING RAPAMYCIN ANALOGS WITH SHORTENED HALF-LIVES"

The hole fillings and/or coatings can also be provided on the endoprosthesis to facilitate the loading or delivery of beneficial agents or drugs, such as therapeutic agents, pharmaceuticals and radiation therapies. As such, the endoprosthetic material and/or holes can be filled and/or coated with a biodegradable material as described above.

Additionally, the biodegradable material can contain a drug or beneficial agent to improve the use of the endoprosthesis. Such drugs or beneficial agents can include antithrombotics, anticoagulants, antiplatelet agents, thrombolytics, antiproliferatives, anti-inflammatories, agents that inhibit hyperplasia, inhibitors of smooth muscle proliferation, antibiotics, growth factor inhibitors, or cell adhesion inhibitors, as well as antineoplastics, antimitotics, antifibrins, antioxidants, agents that promote endothelial cell recovery, antiallergic substances, radiopaque agents, viral vectors having beneficial gene, genes, siRNA, antisense compounds, oligonucleotides, cell permeation enhancers, and combinations thereof. Another example of a suitable berieficial agent is described in US Patent No. 6,015,815 and US Patent No. 6,329,386 entitled "Tetrazole-containing rapamycin analogs with shortened half-lives"

In one embodiment, the endoprosthesis and/or holes can be filled and/or coated with a bio-absorbable material that is configured to be responsive to radio-frequency ("RF") energy or ultrasonic energy. As such, the bio-absorbability of the filling and/or coating can be altered in response to applied RF energy. For example, the absorption rate may be increased or decreased in response to the applied RF energy.

In one embodiment, the endoprosthesis and/or holes can include fillings and/or coatings comprised of polytetrafluorethylene ("PTFE"), expanded PTFE ("ePTFB"), Dacron, woven materials, cut filaments, porous membranes, harvested vessels and/or arteries, or others such materials to form a stent graft prosthesis. Similarly, a medical device, such as a valve, a flow regulator or monitor device, can be attached to the endoprosthesis, such that the endoprosthesis functions as an anchor for the medical device within the body lumen.

In one embodiment, the endoprosthesis and/or holes can be filled and/or coated with an imaging compound or radiopaque material. Preferably, the radiopaque material is filled into the holes of the endoprosthesis. Also, the radiopaque material can be encapsulated within a biocompatible or biodegradable polymer filling and/or coating. For example, the suitable radiopaque material can be platinum, tungsten, silver stainless steel, gold, tantalum, bismuth, barium sulfate, or other similar material. The radiopaque material can be coated on selected surfaces of the endoprosthesis or Filled within selected holes using any of a variety of well-known techniques, including cladding, bonding, adhesion, fusion, deposition, and/or the like.

### V. Endoprosthesis With Dispersed Fibrous or Particulate Material

In addition to the foregoing compositions, a particulate or fibrous material can also be included within the matrix of an endoprosthesis. As such, any of the foregoing compositions can be impregnated and/or encapsulated with a suitable particulate or fibrous material. While the following discussion is directed to fibrous materials, it should be recognized that such particulate materials similar to fibers can be used in conjunction with or in place of the fibrous materials. Thus, an endoprosthetic matrix having a fiber can be modified to include a fiber and/or a particle, and such a modification is within the ordinary practice of one skilled in the art with this disclosure in hand.

The fiber-reinforced materials can range in composition depending on the desired characteristics, and can be included in various amounts, distributions, and orientations. Additionally, the size of the fiber can be modulated from short fibers to long fibers, as well as continuous fibers. The fibers can function in a manner similar to the holes described herein by providing dislocations within the endoprosthetic matrix that inhibit crack formation and/or crack propagation and/or fatigue-induced catastrophic failures. As such, the foregoing discussions related to the use of holes can be applied here to the use of fiber-reinforced endoprosthetic matrices.

Various types of fibers may be used in order to obtain specific characteristics. For example, the endoprosthetic compositions can include naturally occurring organic fibers extracted from hemp, cotton, plant leaves or stems, hardwoods, softwoods, or the like, fibers made from organic polymers, examples of which include polyester and nylon (i.e., polyamide), and/or inorganic fibers, examples of which include glass, graphite, silica, silicates, microglass made alkali resistant using borax, ceramics, carbon fibers, carbides, metals, alloys, and the like. The preferred fibers, for example, include glass fibers, silica nitride, silica carbide, silica nitride, tungsten carbide, and Kevlar; however, other types of synthetic fibers can be preferred.

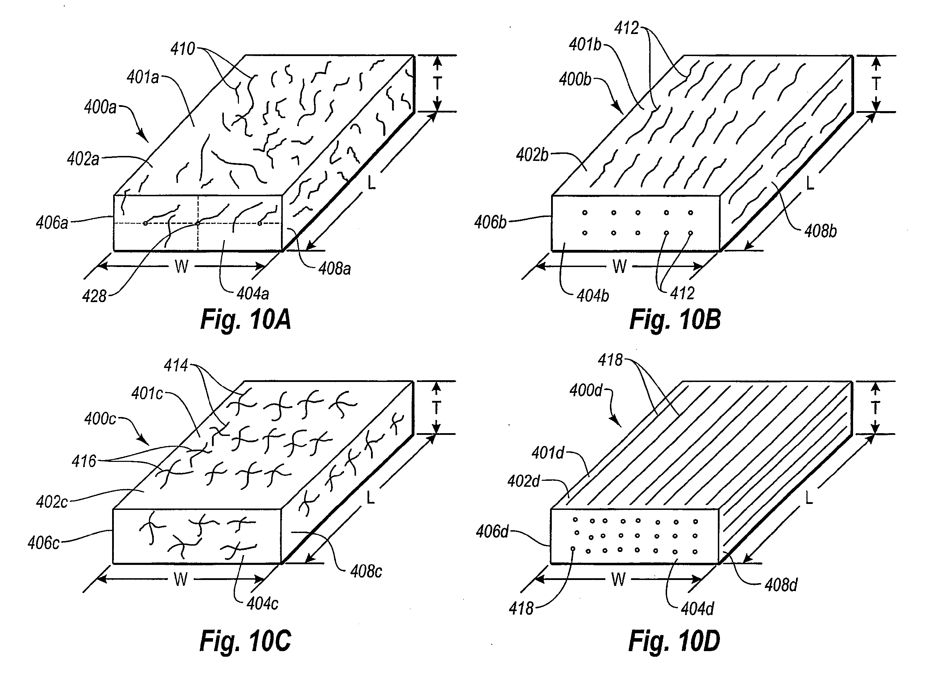

Figure 10A is a schematic diagram illustrating a selected volume of an endoprosthetic element 400a having a-substantially rectangular cross-sectional profile. The element 400a is defined by a top side 402a opposite a bottom side 404a and an inner side 406a opposite an outer side 408a. Accordingly, the element body 401 a between the top-side 402a and the bottom side 404a defines the thickness of the element 400a, and the element body between the inner side 406a and the outer side 408a defines the width of the element. The element 400a is depicted to include a plurality of fibers 410 randomly distributed through the matrix of the body 401 b. Additionally, the fibers 410 can be homogeneously distributed through the matrix of the body 401 a. While the element 400a is defined by a top-side 402a opposite a bottom side 404a and an inner side 406a opposite an outer side 408a, it should be recognized such sides could be swapped by rotating the body 401 a about a longitudinal axis 428 along the length of the element.

Figure 10B is a schematic diagram illustrating a selected volume of an endoprosthetic element 400b having a substantially rectangular cross-sectional profile. The element 400b is defined by a topside 402b opposite a bottom side 404b and an inner side 406b opposite an outer side 408b. Accordingly, the element body 401b between the top-side 402b and the bottom side 404b defines the thickness of the element 400b, and the element body between the inner side 406b and the outer side 408b defines the width of the element. The element 400a is depicted to include a plurality of longitudinal fibers 412 distributed through the matrix of the body 401 a in a longitudinal orientation.

Figure 10C is a schematic diagram illustrating a selected volume of an endoprosthetic element 400c having a substantially rectangular cross-sectional profile. The element 400c is defined by a top side 402c opposite a bottom side 404c and an inner side 406c opposite an outer side 408c. Accordingly, the element body 401c between the top side 402c and the bottom side 404c defines the thickness of the element 400c, and the element body between the inner side 406c and the outer side 408c defines the width of the element. The element 400c is depicted to include a plurality of longitudinal fibers 414 distributed through the matrix of the body 401c in a longitudinal orientation. Additionally, the element 400c is depicted to include a plurality of cross fibers 416 distributed through the matrix of the body 401 c at substantially an orthogonal orientation with respect to the longitudinal fibers 414. While the cross fibers 416 are shown to intersect the longitudinal fibers 414, such intersections are not required.

Figure 10D is a schematic diagram illustrating a selected volume of an endoprosthetic element 400d having a substantially rectangular cross-sectional profile. The element 400d is defined by a top-side 402d opposite a bottom side 404d and an inner side 406d opposite an outer side 408d. Accordingly, the element body 401d between the top side 402d and the bottom side 404d defines the thickness of the element 400d, and the element body between the inner side 406d and the outer side 408d defines the width of the element. The element 400d is depicted to include a plurality of continuous fibers 418 distributed through the matrix of the body 401d in a longitudinal orientation.

The various above-described fibers can be mixed together with the endoprosthetic matrix material by well known techniques. As such, the fibers, metal and/or polymer components can be sufficiently mixed together in order to obtain a composition having fibers homogenously distributed therethrough. It can be appreciated, however, that the fibers may be mixed together such that the fibers are randomly orientated in the endoprosthesis.

### VI. Method of Making Endoprostheses

Various different manufacturing techniques are well known and may be used for fabrication of the endoprosthesis of the present invention. For example, the endoprosthesis can be formed from a hollow tube of suitable material using a known technique, such as by laser cutting, EDM, milling, chemical etching, hydro-cutting, and the like. The shaped structure can be mechanically blasted with a media and then electropolished or otherwise finished to remove burrs and eliminate sharp edges and contaminates. An additional de-scaling process may be performed before electropolishing, wherein the de-scaling process involves the use of an acid bath.

Alternatively, the endoprosthesis can be fabricated from a sheet of suitable material using a similar cutting, milling, or etching technique, and then rolled or bent about a longitudinal axis into the desired shape. If desired, the lateral edges of the structure can be joined together, such as by welding or bonding, to form a closed tubular structure, or the lateral edges can remain unattached to form a coiled, rolled sheet or open tubular structure.

Conversely, a suitable material of construction can be applied selectively to a substrate to define the desired pattern of the endoprosthesis structure, and then the substrate can be removed. Other methods of manufacture also can be used for the endoprosthesis of the present invention, such as by bending toroidal rings or elongate lengths of wire into appropriately shaped members, such as that corresponding to each annular element, and then joining the appropriately shaped members together at connection locations by a welding or bonding technique or the like. If a shape memory material is used, such as nitinol, the fabricated structure can be heat treated on a mandrel or the like using known techniques to establish the desired endoprosthesis shape and dimensions at a predetermined temperature (e.g., when above the austenitic transition temperature). In one method, a tube having a first set of dimensions is fabricated to include endoprosthetic elements and/or holes. This can include obtaining a tube of a suitable material and forming the endoprosthetic elements and/or holes. The tube can then be drawn down to a smaller size of an implantable endoprosthesis. Additional processing well known in the art can then be used to condition the endoprosthesis for use. Thus, multiple implantable endoprostheses can be prepared from the tube having the first set of dimensions.

An additional step of passivation can be performed during the manufacturing stage of the endoprosthesis in order to form a homogeneous oxide layer for corrosion resistance. The passivation process may be performed prior to installation of the markers or it may be performed after installation of radiopaque markers. Alternatively, multiple passivation processes may be performed, once prior to insertion of the markers and again after insertion of the markers.

As originally cut and/or fabricated with holes, the endoprosthesis can correspond to its delivery configuration or to a deployed configuration or a configuration therebetween. Preferably, however, the endoprosthesis is fabricated with a configuration at least slightly larger than the delivery configuration as shown in the planar formats of Figures 2A-2B, for example. In this manner, the endoprosthesis can be crimped or otherwise compressed into its delivery configuration in a corresponding delivery device.

In another method, the endoprosthesis is fabricated from a tube having a diameter corresponding to the deployed configuration. In this manner, the longitudinally-free portions of the annular elements (e.g., elbow or foot not at a connection location) and circumferentially-free portions (e.g., the toe and/or heel portion of the foot extensions) can be maintained within the general cylindrical shape (e.g., diameter) of the endoprosthesis when deployed, so as to avoid such portions from extending radially inwardly when in the deployed-configuration. The endoprosthesis can be designed to match the target vessel in which the endoprosthesis is to be deployed. As previously noted, the geometry of each component of the endoprosthesis or endoprosthetic element, such as the width, thickness, length and shape of the strut elements, crossbars, connectors, elbows, foot portions, ankle portions, toe portions, heel portions and the like can be selected to obtain predetermined expansion, flexibility, foreshortening, coverage scaffolding, and cross-sectional profile characteristics. For example, longer crossbars and/or connectors can promote greater radial expansion or scaffolding coverage. The phase difference or circumferential alignment between adjacent annular elements likewise can be altered to control coverage and flexibility. Similarly, the number and placement of connection locations and, if present, the connectors, between longitudinally-adjacent annular elements can be selected to obtain the desired flexibility of the endoprosthesis. The number of elbows and/or foot extensions between connection locations can also be varied to achieve desired performance characteristics.

### A. Shaping and Hole Formation

An endoprosthetic material can be shaped by various methods as described in more detail below. Such shaping techniques can utilize streams of energy and/or streams of matter in order to impart shapes and/or holes into the endoprosthetic material. The streams of energy include photons, electromagnetic radiation, atomic, and sub-atomic materials, as described above. On the other hand, the streams of matter are considered to include materials larger than atomic scale particles, and can be microscopic or macroscopic in size. In any event, the shaping can be designed to direct a stream of energy or a stream of matter at the endoprosthetic material to form endoprosthetic element and/or holes therein. Also, mechanical drills can be used to drill holes into an endoprosthesis.

In one method, a stream of energy can cut, shape, and/or form holes in the endoprosthetic material by generating heat at the site where the stream intersects the material, as is well known in the art. The thermal interaction can elevate the local temperature to a point which can cut, melt, shape, and/or vaporize portions of the endoprosthetic material from the rest of the material. Accordingly, thestream-cutting apparatus can operate and shape the endoprosthetic material by thermal interactions. As such, any of the thermal processes described herein can be used for thermal-cutting. For example, such thermal interactions can arise from laser beam treatment, laser beam machining, electron beam machining, electrical discharge machining, ion beam machining, and plasma beam machining.

In one arrangement, by knowing the thermal properties of the endoprosthetic material, precise energy requirements can be calculated so that the thermal beam provides the appropriate or minimum energy for melting and/or vaporizing the material without significantly melting undesirable portions of the material. For example, laser beams are a common form of a stream of energy that can be used to shape or form holes in the endoprosthetic material. Additionally, there are instances where a laser is preferred over all other cutting techniques because of the nature of the resulting endoprosthesis as well as the characteristics of the endoprosthetic material.

Accordingly, a laser can be used to cut the endoprosthetic material in circumstances where the power of the laser or the heat generated depend upon the composition of the material to be cut. The ability to vary the laser power is beneficial when different materials are used or the thickness of the same is varied. The laser power can be defined as the rate of which energy is delivered by the beam and is usually measured in units as joules/second or watts. For example, lasers typically used in cutting hardened steel, such as YAG or eximer lasers, can have a power of about 2,000 watts or greater. Some endoprosthetic materials can be shaped with lasers operating below about 2,000 watts, more preferably below about 1,000 watts, and most preferably below about 500 watts. In one configuration, a femto-second laser can be used to shape the material, including forming holes therein. Use of the femto-second laser also reduces the heated affected zone (HAZ) of the material during manufacturing, thereby reducing the localized thermal stress upon the material.

In one arrangement, electrical discharge machining is used to shape endoprosthetic material or form the holes therein. Electrical discharge machining is capable of cutting all types of conductive materials such as exotic metals including titanium, hastaloy, kovar, inconel, hard tool steels, carbides, and the like. In electrical discharge machining, the main interaction between the stream of energy and the endoprosthetic material is thermal, where heat is generated by producing electrical discharges. This can lead to the endoprosthetic material being removed by melting and evaporation. Some examples of electrical discharge machining include wire electron discharge machining, CNC-controlled electrical discharge machining, sinker electrical discharge machining, small hole discharge machining, and the like.

In another arrangement a charged particle beam is used for shaping or forming holes in the endoprosthetic material, wherein charged particle beams are exemplified by electron beams and ion beams. A charged particle beam is a group of electrically-charged particles that have approximately the same kinetic energy and move in approximately the same direction. Usually, the kinetic energies are much higher than the thermal energies of similar particles at ordinary temperatures. The high kinetic energy and the directionality of these charged beams can be useful for cutting and shaping of the endoprosthetic material, as described herein. Additionally, there are some instances where electron beams or ion beams are preferred over other cutting techniques.

In one arrangement, a stream of chemical matter is used in order to shape or form holes in the endoprosthetic material such as by chemical etching or chemical-jet milling. Chemical-jet milling, for example, provides selective and controlled material removal by jet and chemical action. As such, the process is similar to water-jet cutting, which is described in more detail below. In any event, chemical-jet milling can be useful for shaping or forming holes in various types of endoprosthetic materials, which provides intricate shaping capabilities.

In another arrangement, electrochemical shaping is used, and is based on a controlled electrochemical dissolution process similar to chemical-jet milling an endoprosthetic material. As such, the endoprosthetic material can be attached to an electrical source in order to allow an electrical current to assist in the shaping.

In one arrangement, hydro-cutting or water-jet cutting is used to shape and/or form holes in an endoprosthetic material. Hydro-cutting is essentially a water-jet technology that uses the high force and high pressure of a stream of water directed at the endoprosthetic material in order to cut and shape the material as desired. Hydro-cutting can be preferred over some of the other stream-cutting technologies because it can be free of heat, flame, and chemical reactions, and can provide a precise cold shaping technique. Also, heated water with or without being doped with reactive chemicals can also be used. Hydro-cutting is particularly suitable for polymeric endoprosthesis, but can be used for some metal materials when combined with abrasive particles, as described below.

Additionally, hydro-cutting can be enhanced by the introduction of particulate materials into the water feed line. As such, some hydro-cutting techniques utilize garnet or other rigid and strong materials in order to apply an abrasive cutting force along with the force applied by the water itself.

In one arrangement, sandblasting, which fits into the regime of a stream of matter, can be used to shape and/or form holes in an endoprosthetic material by projecting a high energy stream of sand particles at the material. Sandblasting cuts materials similar to hydro-cutting, especially when the water-jet is doped with abrasive particulates. Additionally, various other particulate streams other than sand can be used in the stream-cutting techniques and machinery.

### B. Sintering

One method of making an endoprosthesis includes sintering sinterable particles to provide a sintered article having the shape of the endoprosthesis. Briefly, the sintered body is obtained from a green body prepared by molding a mixture of sinterable particles with or without a binder into the shape of an endoprosthesis or body intermediate. The molded green body may have the shape of the endoprosthetic elements with or without holes. After the green body has been formed in the mold and sintered into a hardened endoprosthesis, the process includes shaping the sintered body with a stream of energy and/or matter in order to obtain a desired shape. In any event, sintering a green body in a mold results in an endoprosthesis that is either ready for use, or requires additional processing or finishing such as shaping and/or hole forming.

In one arrangement, a de-binding process is carried out to remove the binder prior to sintering the green body. As such, the de-binding is performed by heat treatment in an oxidizing or non-oxidizing atmosphere, for instance, under a vacuum or low pressure.

When the green body is sintered, the volume can shrink as the porosity decreases and the density increases, especially when the sinterable particles are held together with a binder. This happens as the majority of the binder is melting and/or evaporating so as to draw the individual sinterable particles closer together. As such, the green body can be fabricated, molded, and/or shaped to be larger than the resultant sintered article in order to accommodate for the volume lost during sintering.

Additionally, the sintered body can be shaped into an endoprosthesis with holes as described herein. Also, the endoprosthesis can be further processed after sintering and/or shaping such as by grinding, sanding, or the like to provide enhanced surface characteristics.

As described herein, one method of manufacturing an endoprosthesis for delivery in a body lumen includes forming a tubular structure having a first material, forming at least one hole in the tubular structure, and shaping the tubular structure into a shape of an endoprosthesis by: cutting a portion of the first material from the tubular structure so as to form a first side of a plurality of interconnected strut elements having the plurality of holes; and cutting a second portion of the first material so as to form a second side of the plurality of interconnected strut elements so as to define the size and shape of the interconnected strut elements, the interconnected strut elements forming an annular element.

The above method can also include forming a first tube, forming the at least one strut element, forming at least one hole, and drawing the first tube into an endoprosthetic-sized tube having the at least one strut element and the at least one hole. Further, the method can include depositing a material, such as a biodegradable material or a material impregnated with a beneficial agent, onto the tubular structure so as to reduce the cross-sectional area of the holes. The deposited material can substantially occlude the holes.

The present invention may be embodied in other specific forms without departing from its essential characteristics. The described embodiments are to be considered in all respects only as illustrative and not restrictive. The scope of the invention is, therefore, indicated by the appended claims rather than by the foregoing description.

## Claims

1. An endoprosthesis for delivery in a body lumen, the endoprosthesis comprising:
at least one strut element (30) having a strut width and a strut thickness, the strut thickness being defined by a first side opposite a second side, the strut element (30) having a plurality of holes (26) extending from the first side to the second side **characterised in that** the plurality of holes are in a random arrangement that extend from a first end (32) to a second end (34) of the strut element (30) to inhibit crack propagation.

2. An endoprosthesis as in claim 1, wherein a line drawn through two of the holes (26) across the strut width partitions the strut element (30) into a first portion and a second portion connected by three connection elements defined by the holes, the holes having a diameter or width such that each of the three connection elements has a sufficient strength so that only two of the connection elements are required to provide structural integrity.

3. An endoprosthesis as in claim 1, wherein the plurality of holes (26) each have a narrowing taper.

4. An endoprosthesis as in claim 1, wherein the plurality of holes (26) each have a widening taper.

5. An endoprosthesis as in claim 1, wherein each of the plurality of holes (26) is shaped as two fluidly coupled parabolic recesses..

6. An endoprosthesis as in claim 1, wherein the plurality of holes (26) are filled with a biodegradable polymer.

7. An endoprosthesis as in claim 1, further comprising at least one radiopaque marking element.

8. An endoprosthesis as in claim 1, wherein a portion of the plurality of holes (26) intersect.

9. An endoprosthesis as in claim 1, wherein the plurality of holes (26) have random orientations with respect to the strut element (30).

10. An endoprosthesis in accordance with any preceding claim, wherein the strut element (30) includes one or more of crossbars (20), elbows (30), foot extensions (40), connectors (60), or combinations thereof.

## Patentansprüche

1. Endoprothese zum Einbringen in ein Körperlumen, wobei die Endoprothese aufweist:
zumindest ein Stegelement (30), das eine Stegbreite und eine Stegdicke aufweist, wobei die Stegdicke durch eine erste Seite gegenüber einer zweiten Seite definiert ist, wobei das Stegelement (30) mehrere Löcher (26) aufweist, die sich von der ersten Seite zur zweiten Seite erstrecken, **dadurch gekennzeichnet, dass** sich die mehreren Löcher in einer zufälligen Anordnung befinden, die sich von einem ersten Ende (32) zu einem zweiten Ende (34) des Stegelements (30) erstreckt, um eine Rissausbreitung zu verhindern.

2. Endoprothese nach Anspruch 1, wobei eine durch zwei der Löcher (26) entlang der Stegbreite gezogene Linie das Stegelement (30) in einen ersten Abschnitt und einen zweiten Abschnitt aufteilt, die durch drei über die Löcher definierte Verbindungselemente verbunden sind, wobei die Löcher einen Durchmesser oder eine Breite aufweisen, sodass jedes der drei Verbindungselemente eine ausreichende Festigkeit aufweist, sodass nur zwei der Verbindungselemente zur Aufrechterhaltung der strukturellen Integrität erforderlich sind.

3. Endoprothese nach Anspruch 1, wobei die mehreren Löcher (26) jeweils verjüngend abgeschrägt sind.

4. Endoprothese nach Anspruch 1, wobei die mehreren Löcher (26) jeweils aufweitend abgeschrägt sind.

5. Endoprothese nach Anspruch 1, wobei jedes der mehreren Löcher (26) als zwei in Fluidverbindung stehende parabolische Ausnehmungen geformt ist.

6. Endoprothese nach Anspruch 1, wobei die mehreren Löcher (26) mit einem biologisch abbaubaren Polymer gefüllt sind.

7. Endoprothese nach Anspruch 1, die zumindest ein röntgendichtes Markierungselement aufweist.

8. Endoprothese nach Anspruch 1, wobei sich ein Teil der mehreren Löcher (26) kreuzt.

9. Endoprothese nach Anspruch 1, wobei die mehreren Löcher (26) in Bezug auf das Stegelement (30) zufällig orientiert sind.

10. Endoprothese nach einem der vorhergehenden Ansprüche, wobei das Stegelement (30) eine oder mehrere Querstege (20), Krümmer (30), Fußausleger (40), Verbinder (60) oder Kombinationen hiervon aufweist.

## Revendications

1. Endoprothèse destinée à être mise en place dans une lumière corporelle, l'endoprothèse comprenant :
au moins un élément d'entretoise (30) ayant une largeur d'entretoise et une épaisseur d'entretoise, l'épaisseur d'entretoise étant définie par un premier côté opposé à un deuxième côté, l'élément d'entretoise (30) ayant une pluralité de trous (26) s'étendant du premier côté au deuxième côté, **caractérisée en ce que** la pluralité de trous sont dans un agencement aléatoire qui s'étend d'une première extrémité (32) à une deuxième extrémité (34) de l'élément d'entretoise (30) pour empêcher la propagation de fissures.

2. Endoprothèse telle que revendiquée dans la revendication 1, dans laquelle une ligne traversant deux trous parmi les trous (26) sur toute la largeur d'entretoise divise l'élément d'entretoise (30) en une première partie et une deuxième partie reliées par trois éléments de connexion définis par les trous, les trous ayant un diamètre ou une largeur tel que/telle que chacun des trois éléments de connexion présente une résistance suffisante de sorte que seulement deux des éléments de connexion soient nécessaires pour fournir une intégrité structurelle.

3. Endoprothèse telle que revendiquée dans la revendication 1, dans laquelle chacun de la pluralité de trous (26) présente un rétrécissement conique.

4. Endoprothèse telle que revendiquée dans la revendication 1, dans laquelle chacun de la pluralité de trous (26) présente un évasement conique.

5. Endoprothèse telle que revendiquée dans la revendication 1, dans laquelle chacun de la pluralité de trous (26) a la forme de deux évidements paraboliques couplés de manière fluidique.

6. Endoprothèse telle que revendiquée dans la revendication 1, dans laquelle la pluralité de trous (26) sont remplis d'un polymère biodégradable.

7. Endoprothèse telle que revendiquée dans la revendication 1, comprenant en outre au moins un élément de marquage radio-opaque.

8. Endoprothèse telle que revendiquée dans la revendication 1, dans laquelle une partie de la pluralité de trous (26) se coupent.

9. Endoprothèse telle que revendiquée dans la revendication 1, dans laquelle la pluralité de trous (26) ont des orientations aléatoires par rapport à l'élément d'entretoise (30).

10. Endoprothèse selon l'une des revendications précédentes, dans laquelle l'élément d'entretoise (30) comporte un(e) ou plusieurs parmi des barres transversales (20), des coudes (30), des extensions formant embase (40), des connecteurs (60), ou des combinaisons de ceux-ci.
